# EUROPÄISCHE PATENTSCHRIFT

(11) **EP 2 957 277 B1**
(45) Veröffentlichungstag und Bekanntmachung des Hinweises auf die Patenterteilung: **26.07.2017**
(21) Anmeldenummer: 15171497.9
(22) Anmeldetag: 10.06.2015
(51) Int. Cl.: A61K 8/46, A61Q 9/04, A61K 8/19

(54) **HAARENTFERNUNGSMITTEL, VERWENDUNG EINES SOLCHEN SOWIE VERFAHREN ZUR ENTFERNUNG VON KÖRPERBEHAARUNG**
HAIR REMOVAL COMPOSITION, USE OF SUCH AND METHOD FOR REMOVING BODY HAIR
PRODUIT DEPILATOIRE, UTILISATION D'UN TEL PRODUIT ET PROCEDE D'ELIMINATION DE LA PILOSITE CORPORELLE

(30) Priorität: 20.06.2014 DE 202014102830 U
(43) Veröffentlichungstag der Anmeldung: 23.12.2015
(73) Patentinhaber: Raad, Michel, 41352 Korschenbroich (DE); Antoni, Manuela, 41352 Korschenbroich (DE)
(72) Erfinder: Raad, Michael, 41352 Korschenbroich (DE)
(74) Vertreter: Albrecht, Ralf

(56) Entgegenhaltungen:
- EP-A1- 1 902 752
- WO-A1-2011/103233
- DE-A1- 2 603 402
- US-A- 5 149 295

## Beschreibung

Die Erfindung betrifft ein Haarentfernungsmittel, dessen Verwendung sowie ein Verfahren zur Entfernung von Körperbehaarung.

Aus dem Stand der Technik sind verschiedene Verfahren sowie Vorrichtungen bekannt, mittels derer ungewünschte Behaarung von verschiedenen Körperstellen entfernt werden kann.

Neben Nass- oder Trockenrasierern kommen Epiliergeräte zum Einsatz, um die Körperoberfläche durch Schneiden bzw. durch Herauszupfen von den Haaren zu befreien. Alternativ werden mit Heißwachs oder geeigneten Zuckerlösungen versehene Streifen verwendet, die auf behaarte Hautstellen aufgetragen werden, um anschließend die Haare durch eine ruckartige Reißbewegung zu entfernen.

Die vorgenannten Haarentfernungsvorrichtungen bzw. -verfahren bringen unterschiedliche Nachteile mit sich. Die Rasur, die eine schmerzfreie Haarentfernung ermöglicht, ist von einem vergleichsweise hohen Verletzungsrisiko begleitet. Selbst wenn es nicht zu Schnittwunden kommt, so können durch die über die Haut fahrenden Klingen Irritationen verursacht werden. Darüber hinaus besteht der Nachteil, dass die Haare vergleichsweise schnell, hart und stoppelig wieder nachwachsen.

Die Epilation, bei der die Haare mit geeigneten elektrisch motorisierten Vorrichtungen herausgezupft werden, ist in der Regel - verglichen mit der Rasur - sehr schmerzhaft. Gleiches gilt für die Haarentfernung mittels Heißwachs oder Zuckerlösung. Diese Verfahren haben außerdem den Nachteil, dass die Haut, da die Haare herausgerissen werden, im Anschluss an die Behandlung stark gereizt ist. Dies kann sich beispielsweise in roten Flecken oder dergleichen niederschlagen, was als erheblicher ästhetischer Nachteil empfunden wird.

Neben den vorgenannten mechanischen Haarentfernungsverfahren stehen ferner chemische Verfahren zur Verfügung. Hierfür wird in der Regel ein Haarentfernungsmittel, welches in Form einer Creme oder eines Schaums bereitsteht, auf die zu behandele Hautoberfläche aufgetragen. In den chemischen Haarentfernungsmitteln befindet sich eine wirksame Komponente, welche in der Lage ist, die Eiweißbindungen des Keratins im Haar aufzuspalten und das Haar unmittelbar ober- bzw. unterhalb der Hautoberfläche aufzulösen. Als wirksame Komponenten kommen dabei insbesondere Thioglycolate zum Einsatz, welche geeignet sind, die Peptid- und Sulfidverbindungen des Keratins im Haar aufzuspalten. Die entsprechende Reaktion findet in einem alkalischen Milieu statt, bei einem pH-Wert von beispielsweise 12.

Aus der EP 1 902 752 A1 beispielsweise ist ein Haarentfernungsmittel in Pulverform vorbekannt, welches 2 bis 8% Calciumthioglycolat, 3 bis 9% Calciumhydroxid, 2 bis 10% Urea, 5 bis 15% Sorbitol, bis zu 1% Parfüm und den Rest Talkum aufweist.

Obwohl derartige Cremes bzw. Schäume in ihrer Verwendung schmerzfrei und komfortabel sind, da diese auf einfache Weise auf die Hautoberfläche aufgetragen und nach einer vorgegebenen Einwirkzeit, zum Beispiel unter der Dusche, einfach wieder abgespült werden können, besteht der Nachteil, dass auch die chemischen Haarentfernungsmittel zu erheblichen Hautirritationen führen können. Dies liegt zum einen daran, dass die für die Haarentfernung benötigten Thioglycolate die Haut stark angreifen. Darüber hinaus befinden sich in den bekannten Haarentfernungsmitteln diverse weitere Inhaltsstoffe, wie Konservierungsmittel, Emulgatoren und dergleichen, welche die Haut auf chemischer Ebene angreifen Schließlich ist die Depilation mittels den bekannten Haarentfernungsmitteln in der Regel von einem unangenehmen Geruch begleitet, was den Komfort der Behandlung deutlich reduziert.

Ausgehend von diesem Stand der Technik ist es eine Aufgabe der vorliegenden Erfindung, ein Haarentfernungsmittel vorzugeben, welches schnell wirkt, schonend sowie schmerzfrei ist und sich bei der Behandlung durch eine vergleichsweise geringe Geruchsbelästigung auszeichnet. Darüber hinaus ist es Aufgabe der vorliegenden Erfindung ein Verfahren anzugeben, welches eine Haarentfernung mit den vorgenannten Vorteilen ermöglicht.

Diese Aufgabe wird gelöst durch ein Haarentfernungsmittel in Pulverform, umfassend oder bestehend aus
- 3 bis 5 Gew.-% Thioglycolat,
- 3 bis 8 Gew.-% Calciumhydroxid,
- 1,5 bis 4 Gew.-% Calciumoxid,
- einem Geruchsabsorber und
- einem Füllstoff.

Das erfindungsgemäße Haarentfernungsmittel zeichnet sich insbesondere durch die Kombination der drei Bestandteile Thioglycolat, Calciumhydroxid und Calciumoxid aus. Es hat sich gezeigt, dass eine Zusammensetzung mit diesen Bestandteilen in den erfindungsgemäß vorgesehenen Mengen besonders geeignet ist, um eine besonders sanfte, komfortable und effiziente Haarentfernung zu gewährleisten.

Dabei dient das Thioglycolat als wirksamer Bestandteil für die Haarentfernung, da es in der Lage ist, das Keratin im Haar aufzuspalten. Mittels des Calciumhydroxids wird einerseits der pH-Wert gesteigert und das Haar aufgeweicht, was die Haarentfernung fördert, und andererseits die Hautoberfläche entfettet, wodurch eine samt-weiche Hautoberfläche erhalten wird-. Das Calciumoxid dient ebenfalls der Steigerung beziehungsweise der Stabilisierung des pH-Wertes. Durch den Anstieg des pH-Wertes werden auch kleinste Hautschuppen auf der Hautoberfläche aufgelöst, was den positiven Nebeneffekt einer Art Peeling hat.

Eine Ausführungsform des erfindungsgemäßen Haarentfernungsmittels zeichnet sich dadurch aus, dass 4,2 bis 4,7 Gew.-%, insbesondere 4,3 bis 4,6 Gew.-%, insbesondere 4,5 Gew.-% Thioglycolat enthalten sind. Ein Thioglycolatanteil in diesem Bereichen hat sich als besonders geeignet erwiesen, um eine schonende Haarentfernung bei gleichzeitig komfortablen Einwirkzeiten, für die das Haarentfernungsmittel auf der Hautoberfläche zu belassen ist, zu ermöglichen. Die Einwirkzeiten können insbesondere im Bereich einiger Minuten liegen.

Was den Bestandteil Calciumhydroxid angeht, kann in Weiterbildung der Erfindung vorgesehen sein, dass dieser 3,5 bis 5 Gew.-%, insbesondere 3,8 bis 4,2 Gew.-%, insbesondere 4 Gew.-% beträgt.

Eine weitere Ausführungsform zeichnet sich dadurch aus, dass 1,8 bis 3 Gew.-%, insbesondere 2 bis 2,5 Gew.-%, insbesondere 2 Gew.-% Calciumoxid enthalten sind.

Um so höher der Calciumhydroxid-Anteil ist, um so schneller steigt auch der pH-Wert, wodurch wiederum eine besonders schnelle Wirkung des Haarentfernungsmittels erzielt wird. Dabei ist jedoch zu beachten, dass eine besonders schnelle Wirkung die Gefahr von Hautreizungen birgt, wenn die vergleichsweise sehr kurze Einwirkzeit überschritten wird. Das erfindungsgemäße Haarentfernungsmittel bietet hier ein optimales Mischverhältnis, mit dem eine komfortabel schnelle Haarentfernung, insbesondere im Bereich von Einwirkzeiten zwischen etwa 4 und 7 Minuten gewährleistet wird, bei gleichzeitig geringem Risiko von Hautschädigungen. Liegt der Calciumhydroxid-Wert im oberen Bereich, so spielt die genaue Einhaltung der Einwirkzeit eine größere Rolle. Es ist grundsätzlich auch möglich, wenngleich nicht nötig, dass die Behandlung mit dem erfindungsgemäßen Haarentfernungsmittel unter professioneller Aufsicht erfolgt. In diesem Fall kann sich die zu behandelnde Person, was das Einhalten der Einwirkzeit angeht, auf die professionelle Hilfe verlassen.

Ein weiterer Bestandteil der erfindungsgemäßen Zusammensetzung ist ein Geruchsabsorber, der dazu dient, die mit der Haarentfernung verbundenen, als unangenehm empfundenen Gerüche zu binden und so eine besonders komfortable Depilation zu ermöglichen.

Als Geruchsabsorber kann beispielsweise Zeolith zum Einsatz kommen, was sich als besonders geeignetes Material herausgestellt hat.

Zeolith absorbiert durch seine große Oberfläche die Gase der Thioglycolsäure, die durch eine Mischung des Thioglycolat mit Wasser entsteht. Als erfindungsgemäßer Geruchsabsorber eignet sich insbesondere Zeolith mit einer Körnung im Bereich von 0 bis 50 Mikrometern, besonders bevorzugt im Bereich von 0 bis 20 Mikrometern. Zeolith ist ein natürliches Material, welches bei der erfindungsgemäßen Verwendung in einem Haarentfernungsmittel keinerlei Risiken von Hautirritationen birgt. Gleichzeitig bindet es die entstehenden Gase, die vom Menschen als sehr unangenehm empfunden werden so gut, dass mit dem erfindungsgemäßen Haarentfernungsmittel eine geruchsfreie Behandlung erfolgen kann.

Der Geruchsabsorber kann in Weiterbildung des erfindungsgemäßen Haarentfernungsmittels 1 bis 3 Gew.-%, insbesondere 1,3 bis 1,7 Gew.-%, insbesondere 1,5 Gew.-% der Zusammensetzung ausmachen. In diesem Bereich liegende Anteile des Geruchsabsorbers ermöglichen eine gute Geruchsbindung.

Schließlich umfasst das erfindungsgemäße Haarentfernungsmittel einen Füllstoff. Dieser kann insbesondere derjenige Bestandteil der Zusammensetzung sein, der den größten Anteil ausmacht. Gemäß einer Ausführungsform der vorliegenden Erfindung macht der Füllstoff 80 bis 90 Gew.-%, insbesondere 88 Gew.-% des erfindungsgemäßen Haarentfernungsmittels aus.

Bei dem Füllstoff kann es sich gemäß einer bevorzugten Ausführungsform der Erfindung um Calciumkarbonat handeln.

Bei Calciumcarbonat handelt es sich um ein sicheres sowie günstiges Produkt, welches nicht chemisch verarbeitet wird. Calciumcarbonat ist sogar als Lebensmittelzusatzstoff - beispielsweise in Speisesalz als Rieselhilfe - zugelassen, was die Unbedenklichkeit dieses Stoffes, der erfindungsgemäß zum Einsatz kommt, unterstreicht.

Das erfindungsgemäße Haarentfernungsmittel wird in Pulverform bereitgestellt. Diese Darreichungsform bietet den großen Vorteil, dass auf eine Vielzahl chemischer Inhaltsstoffe, wie sie beispielsweise in Cremes sowie Schäumen enthalten sind, vollständig verzichtet werden kann. Dies ist darauf zurückzuführen, dass das Haarentfernungsmittel in Pulverform - bis unmittelbar vor der Anwendung, wenn es von einem Benutzer mit Wasser vermischt wird - keine feuchten Bestandteile enthält. So kann auch gänzlich auf jegliche Art von Konservierungsmitteln verzichtet werden. Da das erfindungsgemäße Haarentfernungsmittel gegenüber dem Stand der Technik mit deutlich weniger Bestandteilen und insbesondere ohne Konservierungsmittel auskommt, ist die Gefahr von Hautirritationen und Allergien deutlich reduziert. Die Haarentfernung wird besonders schonend und angenehm.

In besonders vorteilhafter Ausgestaltung weist das erfindungsgemäße Haarentfernungsmittel in Pulverform ausschließlich die Inhaltsstoffe Thioglycolat, Calciumhydroxid, Calciumoxid, einen Geruchsabsorber sowie einen Füllstoff auf.

Es versteht sich dabei, dass diese Bestandteile jeweils in einer Menge enthalten sind, welche in den zuvor beschriebenen Bereichen liegen, wobei dem Fachmann klar ist, dass, wenn die Mengen dieser fünf Bestandteile insgesamt 100 % des erfindungsgemäßen Haarentfernungsmittels ausmachen, die Mengen der einzelnen Bestandteile entsprechend aufeinander abgestimmt werden müssen.

Eine konkrete Zusammensetzung, die sich als besonders geeignet für eine sichere, sanfte und effiziente Haarentfernung erwiesen hat, ist gegeben durch:
- 4,5 Gew.-% Calcium-Thioglycolat,
- 4 Gew.-% Calciumhydroxid,
- 2 Gew.-% Calciumoxid,
- 1,5 Gew.-% Zeolith und
- 88 Gew.-% Calciumcarbonat.

In bevorzugter Ausführungsform des erfindungsgemäßen Haarentfernungsmittels kann ferner vorgesehen sein, dass es sich bei dem Thioglycolat um Calcium-Thioglycolat, insbesondere um Calcium-Thioglycolat-Trihydrat handelt, was sich als besonders geeignete wirksame Komponente zur Haarentfernung erwiesen hat.

Ein weiterer Aspekt der vorliegenden Erfindung ist ferner die Verwendung des zuvor beschriebenen Haarentfernungsmittel in Pulverform zur Entfernung menschlicher Körperbehaarung, insbesondere der Arm-, Bein-, Bauch-, Rücken- sowie Intimbehaarung.

Die vorgenannte Aufgabe wird des Weiteren gelöst durch ein Verfahren zur Entfernung von Körperbehaarung, insbesondere zur Entfernung von Haaren an Armen, Beinen, Bauch, Rücken sowie im Intimbereich, umfassend die Schritte:
- Bereitstellen des zuvor beschriebenen erfindungsgemäßen Haarentfernungsmittels in Pulverform in einer vorgegebenen Menge.
- Vermischen der vorgegebenen Menge des Haarentfernungsmittels in Pulverform mit einer vorgegebenen Menge Wasser, um eine Haarentfernungsmittel in Pastenform bzw. Cremeform zu erhalten.
- Auftragen des erhaltenen Haarentfernungsmittels in Pastenform bzw. Cremeform auf die Köperoberfläche.
- Abwarten einer vorgegebenen Einwirkzeit.
- Entfernen des Haarentfernungsmittels von der Hautoberfläche, insbesondere mittels Wasser und kreisenden Handbewegungen.

Für die Haarentfernung wird das erfindungsgemäße Haarentfernungsmittel in Pulverform von einem Benutzer, der sich enthaaren möchte, in einem geeigneten Verhältnis mit Wasser vermischt, um eine Paste bzw. Creme zu erhalten. Diese wird auf die Körperfläche an der Stelle beziehungsweise den Stellen, an welchen die Entfernung von Haaren gewünscht ist, zum Beispiel unter Verwendung eines Spatels, aufgetragen. Da das Haarentfernungsmittel nach dem Hinzufügen des Wassers eine pasten- bzw. cremeartige Konsistenz aufweist, kann es auch einfache Weise auf die Hautoberfläche aufgetragen werden. Der Benutzer wartet im Anschluss eine vorgegebene Einwirkzeit ab. Diese beträgt in der Regel einige Minuten. Zum Beispiel kann die Einwirkzeit im Bereich von 4 bis 10 Minuten liegen, wobei diese je nach Beschaffenheit der zu entfernenden Haare variiert. Zum Schluss entfernt der Benutzer die Paste bzw. Creme, insbesondere unter zur Hilfenahme von etwas Wasser und durch kreisende Handbewegungen.

In vorteilhafter Ausgestaltung des erfindungsgemäßen Verfahrens ist vorgesehen, dass im zweiten Schritt das Haarentfernungsmittel in Pulverform in einem Verhältnis im Bereich von 10:3 bis 10:5, insbesondere in einem Verhältnis von 10:4 mit Wasser vermischt wird.

Diese Verhältnisse haben sich als besonders geeignet erwiesen, um eine Paste bzw. Creme mit einer Konsistenz zu erhalten, die sich besonders komfortabel und gleichmäßig von dem Benutzer auf der Hautoberfläche auftragen lässt und gleichzeitig eine effiziente Entfernung der Haare gewährleistet. Dafür werden beispielsweise 100g des erfindungsgemäßen Haarentfernungsmittel in Pulverform bereitgestellt und mit 30 bis 50 ml, insbesondere mit 40 ml Wasser vermischt.

Weitere Merkmale und Vorteile der vorliegenden Erfindung werden anhand der nachfolgenden Beschreibung eines Haarentfernungsmittels in Pulverform gemäß einer Ausführungsform der vorliegenden Erfindung sowie dessen Verwendung deutlich.

Ein Benutzer, der seine Achseln von unerwünschter Behaarung befreien möchte, kann hierfür 50 gr. eines Haarentfernungsmittels in Pulverform mit der folgenden beispielhaften Zusammensetzung verwenden:
- 4,5 Gew.-% Thioglycolat,
- 4 Gew.-% Calciumhydroxid,
- 2 Gew.-% Calciumoxid,
- 1,5 Gew.-% Zeolith und
- 88 Gew.-% Calciumcarbonat.

Für die Entfernung der Achselhaare stellt der Benutzer zunächst eine Paste bzw. Creme aus dem pulverförmigen Haarentfernungsmittel her, indem er die 50 gr. des Pulvers in einem geeigneten Gefäß mit etwa 20 ml Wasser vermisch. Dabei kann er eine Spatel zu Hilfe nehmen. Anschließend trägt er die erhaltene Paste bzw. Creme auf die Achseln auf und zwar derart, dass die Körperhaare vollständig bedeckt sind. Er wartet dann eine Einwirkzeit von etwa 4 bis 7 Minuten ab, wobei die Zeit mit dem Haartyp, der Haardichte und der Haarlänge variiert.

Zwischendurch kann der Benutzer mit einem angefeuchteten Finger und kreisenden Bewegungen probieren, ob sich die Haare bereits von den Achseln lösen lassen. Ist dies der Fall, spätestens jedoch nach den 7 Minuten maximaler Einwirkzeit, kann er mit etwas lauwarmen Wasser und kreisenden Handbewegungen die Haare unkompliziert von der behandelten Hautstelle lösen.

Anschließend sollten die auf diese Weise behandelten, nun haarfreien Achseln sorgfältig mit Wasser abgeduscht werden.

Im Ergebnis erhält der Benutzer vollständig von Haaren befreite Achseln, wobei er während der Behandlung mit dem erfindungsgemäßen Haarentfernungsmittel keine Schmerzen hat und auch keiner unangenehmen Geruchsbelästigung ausgesetzt ist. Da das erfindungsgemäße Haarentfernungsmittel besonders sanft ist und keine unnötigen, schädlichen Inhaltsstoffe aufweist, treten auch keine Hautirritationen auf und das Risiko einer allergischen Reaktion wird auf ein Minimum reduziert.

## Patentansprüche

1. Haarentfernungsmittel in Pulverform, umfassend oder bestehend aus
- 3 bis 5 Gew.-% Thioglycolat,
- 3 bis 8 Gew.-% Calciumhydroxid,
- 1,5 bis 4 Gew.-% Calciumoxid,
- einem Geruchsabsorber und
- einem Füllstoff.

2. Haarentfernungsmittel nach Anspruch 1 **dadurch gekennzeichnet, dass** 4,2 bis 4,7 Gew.-%, insbesondere 4,3 bis 4,6 Gew.-%, insbesondere 4,5 Gew.-% Thioglycolat enthalten sind.

3. Haarentfernungsmittel nach Anspruch 1 oder 2, **dadurch gekennzeichnet, dass** 3,5 bis 5 Gew.-%, insbesondere 3,8 bis 4,2 Gew.-%, insbesondere 4 Gew.-% Calciumhydroxid enthalten sind.

4. Haarentfernungsmittel nach einem der vorhergehenden Ansprüche, **dadurch gekennzeichnet, dass** 1,8 bis 3 Gew.-%, insbesondere 2 bis 2,5 Gew.-%, insbesondere 2 Gew.-% Calciumoxid enthalten sind.

5. Haarentfernungsmittel nach einem der vorhergehenden Ansprüche, **dadurch gekennzeichnet, dass** 80 bis 90 Gew.-%, insbesondere 88 Gew.-% Füllstoff enthalten sind.

6. Haarentfernungsmittel nach einem der vorhergehenden Ansprüche, **dadurch gekennzeichnet, dass** 1 bis 3 Gew.-%, insbesondere 1,3 bis 1,7 Gew.-%, insbesondere 1,5 Gew.-% Geruchsabsorber enthalten sind.

7. Haarentfernungsmittel nach einem der vorhergehenden Ansprüche, **dadurch gekennzeichnet, dass** als Füllstoff Calciumcarbonat enthalten ist.

8. Haarentfernungsmittel nach einem der vorhergehenden Ansprüche, **dadurch gekennzeichnet, dass** als Geruchsabsorber Zeolith enthalten ist.

9. Haarentfernungsmittel in Pulverform bestehend aus
- 4,5 Gew.-% Calcium-Thioglycolat,
- 4 Gew.-% Calciumhydroxid,
- 2 Gew.-% Calciumoxid,
- 1,5 Gew.-% Zeolith und
- 88 Gew.-% Calciumcarbonat.

10. Haarentfernungsmittel nach Anspruch 8 oder 9, **dadurch gekennzeichnet, dass** als Geruchsabsorber Zeolith mit einer Körnung im Bereich von 0 bis 50 Mikrometer, insbesondere im Bereich von 0 bis 20 Mikrometern enthalten ist.

11. Haarentfernungsmittel nach einem der vorhergehenden Ansprüche, **dadurch gekennzeichnet, dass** es sich bei dem Thioglycolat um Calcium-Thioglycolat, insbesondere um Calcium-Thioglycolat-Trihydrat handelt.

12. Verwendung eines Haarentfernungsmittels nach einem der vorhergehenden Ansprüche zur Entfernung menschlicher Körperbehaarung, insbesondere der Arm-, Bein-, Bauch-, Rücken- sowie Intimbehaarung.

13. Verfahren zur Entfernung von Körperbehaarung, insbesondere zur Entfernung von Haaren an Armen, Beinen, Bauch, Rücken sowie im Intimbereich, umfassend die Schritte:
- Bereitstellen eines Haarentfernungsmittels in Pulverform nach einem der Ansprüche 1 bis 11 in einer vorgegebenen Menge.
- Vermischen der vorgegebenen Menge des Haarentfernungsmittels in Pulverform mit einer vorgegebenen Menge Wasser, um eine Haarentfernungsmittel in Pastenform bzw. Cremeform zu erhalten.
- Auftragen des erhaltenen Haarentfernungsmittels in Pastenform bzw. Cremeform auf die Körperoberfläche.
- Abwarten einer vorgegebenen Einwirkzeit.
- Entfernen des Haarentfernungsmittels von der Hautoberfläche, insbesondere mit Wasser und durch kreisende Handbewegungen.

14. Verfahren nach Anspruch 13, **dadurch gekennzeichnet, dass** im zweiten Schritt das Haarentfernungsmittel in Pulverform in einem Verhältnis im Bereich von 10:3 bis 10:5, insbesondere in einem Verhältnis von 10:4 mit Wasser vermischt wird.

## Claims

1. A depilatory in powder form, comprising or made up of
- 3 to 5 % by weight thioglycolate,
- 3 to 8 % by weight calcium hydroxide,
- 1.5 to 4 % by weight calcium oxide,
- an odour absorber and
- a filler.

2. The depilatory according to claim 1, **characterised in that** 4.2 to 4.7 % by weight, in particular 4.3 to 4.6 % by weight, in particular 4.5 % by weight thioglycolate are included.

3. The depilatory according to claim 1 or 2, **characterised in that** 3.5 to 5 % by weight, in particular 3.8 to 4.2 % by weight, in particular 4 % by weight calcium hydroxide are included.

4. The depilatory according to any of the preceding claims, **characterised in that** 1.8 to 3 % by weight, in particular 2 to 2.5 % by weight, in particular 2 % by weight calcium oxide are included.

5. The depilatory according to any of the preceding claims, **characterised in that** 80 to 90 % by weight, in particular 88 % by weight filler are included.

6. The depilatory according to any of the preceding claims, **characterised in that** 1 to 3 %, in particular 1.3 to 1.7 % by weight, in particular 1.5 % by weight odour absorber are included.

7. The depilatory according to any of the preceding claims, **characterised in that** calcium carbonate is included as a filler.

8. The depilatory according to any of the preceding claims, **characterised in that** zeolite is included as an odour absorber.

9. A depilatory in powder form made up of
- 4.5 % by weight calcium thioglycolate,
- 4 % by weight calcium hydroxide,
- 2 % by weight calcium oxide,
- 1.5 % by weight zeolite and
- 88 % by weight calcium carbonate.

10. The depilatory according to claim 8 or 9, **characterised in that** zeolite with a grain size in the range of 0 to 50 micrometres, in particular in the range of 0 to 20 micrometres, is included as an odour absorber.

11. The depilatory according to any of the preceding claims, **characterised in that** the thioglycolate is calcium thioglycolate, in particular calcium thioglycolate trihydrate.

12. The use of a depilatory according to any of the preceding claims for removing human body hair, in particular hair on the arms, legs, stomach and back as well as pubic hair.

13. A method for the removal of body hair, in particular for the removal of hair on the arms, legs, stomach, back and in the intimate area, including the steps:
- providing a depilatory in powder form according to any of claims 1 to 11 in a pre-specified amount,
- mixing the pre-specified amount of depilatory in powder form with a pre-specified amount of water in order to obtain a depilatory in paste form or in cream form,
- applying the depilatory that is obtained in paste form or cream form to the surface of the body,
- waiting for a pre-specified time for the depilatory to take effect,
- removing the depilatory from the surface of the skin, in particular with water and by circling hand movements.

14. The method according to claim 13, **characterised in that** in the second step, the depilatory in powder form is mixed with water at a ratio in the range of 10:3 to 10:5, in particular at a ratio of 10:4.

## Revendications

1. Produit dépilatoire sous forme de poudre, comprenant ou consistant en
- 3 à 5% en poids de thioglycolate,
- 3 à 8% en poids d'hydroxyde de calcium,
- 1,5 à 4% en poids d'oxyde de calcium,
- un absorbeur d'odeurs et
- un agent de remplissage.

2. Produit dépilatoire selon la revendication 1, **caractérisé en ce que** 4,2 à 4,7% en poids, notamment 4,3 à 4,6%en poids, notamment 4,5% en poids de thioglycolate sont contenus.

3. Produit dépilatoire selon la revendication 1 ou 2, **caractérisé en ce que** 3,5 à 5% en poids, notamment 3,8 à 4,2% en poids, notamment 4% en poids d'hydroxyde de calcium sont contenus.

4. Produit dépilatoire selon l'une quelconque des revendications précédentes, **caractérisé en ce que** 1,8 à 3% en poids, notamment 2 à 2,5% en poids, notamment 2% en poids d'oxyde de calcium sont contenus.

5. Produit dépilatoire selon l'une quelconque des revendications précédentes, **caractérisé en ce que** 80 à 90% en poids, notamment 88% en poids d'agent de remplissage sont contenus.

6. Produit dépilatoire selon l'une quelconque des revendications précédentes, **caractérisé en ce que** 1 à 3% en poids, notamment 1,3 à 1,7% en poids, notamment 1,5% en poids d'absorbeur d'odeurs sont contenus.

7. Produit dépilatoire selon l'une quelconque des revendications précédentes, **caractérisé en ce que** du carbonate de calcium est contenu en tant qu'agent de remplissage.

8. Produit dépilatoire selon l'une quelconque des revendications précédentes, **caractérisé en ce que** de la zéolithe est contenue en tant qu'absorbeur d'odeurs.

9. Produit dépilatoire sous forme de poudre consistant en
- 4,5% en poids de thioglycolate de calcium
- 4% en poids de d'hydroxyde de calcium
- 2% en poids d'oxyde de calcium,
- 1,5% en poids de zéolithe et
- 88% en poids de carbonate de calcium.

10. Produit dépilatoire selon la revendication 8 ou 9, **caractérisé en ce que** de la zéolithe ayant une granularité située dans la plage de 0 à 50 micromètres, notamment dans la plage de 0 à 20 micromètres, est contenue en tant qu'absorbeur d'odeurs.

11. Produit dépilatoire selon l'une quelconque des revendications précédentes, **caractérisé en ce que** le thioglycolate est du thioglycolate de calcium, notamment du thioglycolate de calcium trihydraté.

12. Utilisation d'un produit dépilatoire selon l'une quelconque des revendications précédentes, destiné à retirer la pilosité sur l'être humain, notamment la pilosité des bras, des jambes, du dos et des parties intimes.

13. Procédé destiné à retirer la pilosité, notamment pour retirer les poils sur les bras, les jambes, le ventre, le dos ainsi que dans les parties intimes, comprenant les étapes:
- Préparation d'un produit dépilatoire sous forme de poudre selon l'une quelconque des revendications 1 à 11, dans une quantité prédéfinie.
- Mélange de la quantité prédéfinie du produit dépilatoire sous forme de poudre avec une quantité définie d'eau afin d'obtenir un produit dépilatoire sous forme de pâte ou de crème.
- Application sur la surface du corps du produit dépilatoire obtenu sous forme de pâte ou de crème.
- Attente d'un temps d'action prédéfini.
- Retrait du produit dépilatoire sur la surface cutanée, notamment à l'aide d'eau et par des mouvements circulaires de la main.

14. Procédé selon la revendication 13, **caractérisé en ce qu'**à la deuxième étape, le produit dépilatoire sous forme de poudre est mélangé avec de l'eau dans un rapport compris dans la plage de 10:3 à 10:5, notamment dans un rapport de 10:4.
